(19) Europäisches Patentamt
European Patent Office
Office européen des brevets

(11) **EP 3 466 487 A1**

(12) **EUROPEAN PATENT APPLICATION**

(43) Date of publication:
**10.04.2019 Bulletin 2019/15**

(51) Int Cl.:
**A61N 5/10** (2006.01)

(21) Application number: **17194553.8**

(22) Date of filing: **03.10.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **ISOLA, Alfonso Agatino
5656 AE Eindhoven (NL)**
• **NEUKIRCHEN, Christoph
5656 AE Eindhoven (NL)**
• **HAUTVAST, Guillaume Leopold Theodorus
Frederik
5656 AE Eindhoven (NL)**
• **SENDEN, Dave
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **ROBUSTNESS EVALUATION OF BRACHYTHERAPY TREATMENT PLAN**

(57) The invention relates to a system and a method for assisting in planning a radiation therapy treatment, the treatment being delivered on the basis of a treatment plan including error-prone parameter values of first treatment parameters. In the system, a robustness evaluation unit (10) obtains planned parameter values of the first treatment parameters, generates perturbed parameter configurations including perturbed values of the first treatment parameters, the perturbed parameter values deviating from the planned parameter values by possible error values of the first treatment parameters occurring during the treatment, and estimates for each perturbed parameter configuration a radiation dose distribution resulting from a treatment delivered on the basis of the perturbed parameter configuration and/or determines the treatment plan on the basis of the perturbed parameter configurations.

FIG. 1

**Description**

FIELD OF THE INVENTION

The invention generally relates to a planning of a radiation therapy treatment, particularly a brachytherapy treatment. More specifically, the invention relates to a system, a method and a computer program for assisting in planning a treatment plan for a radiation therapy treatment of a target structure within a region of a patient body.

BACKGROUND OF THE INVENTION

In radiation therapy, target structures, such as tumors, within patients' bodies are treated by means radioactive or electromagnetic radiation or ultrasound waves in order to control growth of or kill cancer cells. At the same time, the treatment is delivered in such a way that the radiation or thermal dose delivered to surrounding healthy structures, which are usually also referred to as organs at risk (OARs), is as low as possible.

One exemplary radiation therapy procedure is the so called temporary brachytherapy in which an applicator is used to place one or more radioactive radiation source(s) within the treatment region for a defined short time interval (usually referred to as dwell time) in order to apply a defined radiation dose particularly to the tumor cells.

The treatment parameters for controlling the treatment are included in a treatment plan, which is generated in a planning system. In order to determine the treatment plan, a so-called inverse planning procedure maybe carried out. In such a procedure, the target structure and surrounding OARs are identified and treatment goals are specified in accordance with a medical prescription for the patient, where the treatment goals specify requirements for the dose delivered to the target structure and the OARs. Then, an optimization process is carried out to find the treatment parameters that result in an optimized radiation dose distribution that fulfills the treatment goals.

In brachytherapy, the treatment parameters include the positions of the radiation sources in the treatment region, which are also referred to as dwell positions, and the related dwell times. However, in practical implementations, the actual values of these parameters deviate from the values included in the treatment plan by error values resulting from inaccuracies in the determination of the parameters and/or in setting the parameters to the values specified in the treatment plan. For instance, in case of the dwell positions, these inaccuracies result from errors occurring in the determination of related position information. This position information may be determined on the basis of on an image of the treatment region and/or using an automatic tracking arrangement, and in both cases the determined position information may include a certain error. In case of the dwell times, inaccuracies particularly result during the automatic or manual handling of the radiation source(s) that may not allow setting the dwell times precisely to their values included in the treatment plan.

As a result of these errors of the actual parameter values, the distribution of the radiation dose actually delivered to the treatment region during the treatment may deviate from the optimized dose distribution resulting from the treatment parameter values included in the treatment plan. Depending on the actual values of the treatment parameters, the treatment goals may still be sufficiently fulfilled despite this deviation or the deviation may be such that the treatment goals are not fulfilled.

In view of this, it would be desirable to estimate prior to the delivery of the treatment whether potential error values of the treatment parameters occurring during the treatment could result in a delivered radiation dose distribution that does not fulfill the treatment goals. On the basis of such an estimation, it could then be decided whether the treatment should delivered on the basis of a given treatment plan or whether the treatment plan should be discarded due to an increased risk that the treatment goals will not be met as a result of the treatment. Moreover, it would be desirable to be able to generate treatment plans that are more robust against inaccuracies of the aforementioned type.

SUMMARY OF THE INVENTION

Therefore, it is an object of the present invention to allow for an estimation of the robustness of a treatment plan against inaccuracies in the determination of a parameter values and/or in setting the treatment parameters to the values indicated in the treatment plan. It is a further object of the invention to allow for a generation of a treatment plan that is more robust against such inaccuracies.

In one aspect, the invention suggests a system for assisting in planning a radiation therapy treatment of a target structure in a region of a patient body, the treatment being delivered on the basis of a treatment plan including error-prone parameter values of first treatment parameters for controlling the delivery of the radiation. The system comprises a robustness evaluation unit configured to

- obtain planned parameter values of the first treatment parameters,
- generate perturbed parameter configurations including perturbed parameter values of the first treatment parameters, the perturbed parameter values deviating from the planned parameter values by possible error values of the first treatment parameters occurring during the treatment, and
- estimate for each perturbed parameter configuration a radiation dose distribution resulting from a treatment delivered on the basis of the perturbed parameter configuration and/or
- determine the treatment plan on the basis of the perturbed parameter configurations.

The planned values of the first treatment parameters may be determined as a result of treatment planning process. This also includes that the first treatment parameter are set in accordance with values determined in a planning process and that the planned values of the first treatment parameters correspond to the values measured upon having set the first treatment parameters.

The planned parameter values of the first treatment parameters may particularly be included in a preliminary treatment plan generated for the radiation therapy treatment. By estimating radiation dose distributions corresponding to the perturbed parameter configurations, it is possible to simulate error scenarios that could occur as a result of inaccuracies in the determination of the values of the first treatment parameters and/or in setting the first treatment parameters to their planned values included in the preliminary treatment plan. Here, each perturbed parameter configuration may correspond to one error scenario and includes a set of perturbed values for the first treatment parameters. On this basis, it can particularly be estimated whether inaccuracies can result in a delivered radiation dose distribution that does not fulfill the treatment goals or whether the preliminary treatment plan is sufficiently robust against such inaccuracies. The latter may particularly be the case if the treatment goals can still be fulfilled on the basis of possible perturbed values of the first treatment parameter.

Moreover, the robustness evaluation unit may be configured to generate the (final) treatment plan for the radiation therapy treatment on the basis of the perturbed parameter configurations. By taking the perturbed parameter configurations into consideration in the generation of the treatment plan, it is possible to provide a treatment plan that is more robust against inaccuracies in the determination of parameter values of the first treatment parameters and/or in setting the first treatment parameters to the values indicated in the treatment plan.

In one embodiment of the invention, the radiation therapy treatment is a brachytherapy treatment. In this embodiment, the radiation is emitted by radiation sources located within the patient body at dwell positions during dwell times, where each radiation source is particularly associated with a related dwell position and a related dwell time, and where the first treatment parameters correspond to the dwell positions and/or the dwell times.

In a further embodiment of the invention, the perturbed parameter values deviate from the planned parameter values by a predetermined maximum amount or less. For instance, the predetermined maximum amount may correspond to the maximum or mean error in the determination of the parameter values of the first treatment parameters and/or in the setting of the first treatment parameters to desired values. Thus, if the first treatment parameters correspond to dwell positions of radiation sources, the predetermined maximum amount may correspond to a position error occurring in the determination of the dwell positions. Accordingly, a perturbed dwell position may be located in a region surrounding a related planned dwell position, where a radius of the region may correspond to the position error.

In a further embodiment of the invention, the perturbed parameter values are generated randomly. In particular, the perturbed parameter values may be randomly generated within the limits defined by the aforementioned predetermined amounts. By generating perturbed values randomly, more realistic error scenarios can be generated.

In one embodiment of the invention, the planned values of the first treatment parameters are included in a preliminary treatment plan and each of the radiation dose distributions estimated by the robustness evaluation unit for a perturbed parameter configuration results from the preliminary treatment plan if the planned values of the first treatment parameter are replaced by the perturbed values of the first treatment parameters included in the respective perturbed parameter configuration. As already said above, it is possible in this embodiment to evaluate the robustness of the preliminary treatment plan against inaccuracies in the determination of the parameter values of the first treatment parameters and/or in the setting of the first treatment parameters to the planned parameter values.

In a further embodiment, the system is configured to visualize the radiation dose distributions estimated for the perturbed parameter configurations to a user of the system and/or to determine at least one statistical feature from the radiation dose distributions. On the basis of the visualizations of the radiation dose distributions, the user may particularly evaluate the robustness of the preliminary treatment plan, e.g. by determining whether the radiation dose distributions are acceptable (in this case, the preliminary treatment plan may be considered to be sufficiently robust) or not (in this case, the preliminary treatment plan may be considered to lack sufficient robustness). In addition or as an alternative, the robustness of the preliminary treatment plan may be evaluated on the basis of the statistical features. This evaluation may be carried out automatically and/or by the user of the system. Examples of statistical features of a radiation dose distribution, which may be determined in the system, include a dose volume histogram curve and/or parameters derived from such a curve.

In one embodiment of the invention, which relates to a brachytherapy treatment, the planned values of the first treatment parameters are included in a preliminary treatment plan, which corresponds to a planned radiation dose distribution, and the robustness evaluation unit is configured to estimate the radiation dose distribution for each perturbed parameter configuration by calculating dose values for volume elements of the treatment region in regions surrounding the dwell positions, the sizes of the regions being determined on the basis of the dwell times, and by taking dose values for volume elements of the treatment region outside the regions from the planned radiation dose distribution. Hereby, the computational complexity of the determination of the radiation dose distributions is reduced, because a large number of dose values only has to be calculated once. Moreover, the planned radiation dose distribution is typically determined in the process of generating the preliminary treatment plan and, thus, is already available in the system when the robustness of the preliminary treatment plan is evaluated.

Moreover, as said above, the system may be configured to generate the treatment plan on the basis of the perturbed parameter configurations. In a related embodiment, the treatment plan further includes parameter values of second treatment parameters and the robustness evaluation unit is configured to generate the treatment plan by optimizing the parameters value of the second treatment parameters on the basis of the perturbed parameter configurations. In such a way, a treatment plan can be generated which is more robust against inaccuracies in the determination of the parameters values of the first treatment parameters and/or in the setting of the first treatment parameter values to the planned values.

In a further related embodiment, the system is configured to generate a treatment plan of a brachytherapy treatment. In this embodiment, the radiation may be emitted by radiation sources located within the patient body at a dwell positions during a dwell times, the first treatment parameters may correspond to the dwell positions and the second treatment parameters may correspond to the dwell times. The treatment plan generated in the system may include parameter values of the dwell times optimized on the basis of the perturbed parameter configurations and planned values of the dwell positions. These values may be determined on the basis of a predetermined heuristic procedure, for example.

In one embodiment, the robustness evaluation unit is configured to generate a set of cost functions comprising one cost function for each of at least some of the perturbed parameter configurations and to generate the treatment plan on the basis of the set of cost functions. The cost functions may be generated on the basis of the treatment goals of the radiation therapy treatment. In a related embodiment, the robustness evaluation unit is configured to generate the treatment plan by determining the

$$\min_{t}\left[\max_{k} F_{k}(t)\right]$$

where $F_k$ denotes the cost function for the $k$-th perturbed parameter configuration and $t$ denotes a set of parameter values of the second treatment parameters. In this embodiment, the robustness evaluation unit seeks to minimize the maximum of all cost functions. Hereby, a robust treatment plan can efficiently be determined on the basis of the perturbed parameter configurations.

In a further embodiment, the generated treatment plan only includes parameter values of a subset of the first treatment parameters and the subset does not include values of one or more first treatment parameters having a greater influence on the radiation dose distribution corresponding to the treatment plan than other first treatment parameters. The first treatment parameters having a greater influence on the radiation distribution have a larger potential to affect the robustness of the treatment plan against inaccuracies in the determination of the parameter values of the first treatment parameters and/or in the setting of the first treatment parameters to their planned values values. If these treatment parameters are excluded from the treatment, the likelihood of generating a more robust treatment plan may therefore be increased. In a related embodiment, in which a treatment plan for a brachytherapy treatment is generated, the influence of a dwell position of a radiation source on the radiation dose distribution is particularly determined on the basis of the dwell time of the radiation source.

In accordance with a further aspect, the invention suggests a method for assisting in planning a radiation therapy treatment of a target structure in a region of a patient body, the treatment being delivered on the basis of a treatment plan including error-prone parameter values of first treatment parameters for controlling the delivery of the radiation. The method comprises:

- obtaining planned parameter values of the first treatment parameters,
- generating perturbed parameter configurations including perturbed values of the first treatment parameters, the perturbed parameter values deviating from the planned parameter values plan by possible error values of the first treatment parameters occurring during the treatment,
- estimating for each perturbed parameter configuration a radiation dose distribution resulting from a treatment delivered on the basis of the perturbed parameter configuration and/or
- determining the treatment plan on the basis of the perturbed parameter configurations.

In accordance with a further aspect, the invention suggests a computer program comprising program code for instructing a computer device to perform the method when the program code is executed in the computer device.

It shall be understood that the system of claim 1, the method of claim 14 and the computer program of claim 15, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically and exemplarily shows components of a brachytherapy system,
Fig. 2 schematically and exemplarily shows sets of dose volume histogram curves determined for a set of perturbed parameter configurations,
Fig. 3 schematically and exemplarily shows influence regions of radiations sources located at certain dwell positions, and
Fig. 4 schematically and exemplarily illustrates steps carried out in embodiments of the brachytherapy system in order to analyze the robustness of a preliminary treatment plan and/or to generate a robust treatment plan.

DETAILED DESCRIPTION OF EMBODIMENTS

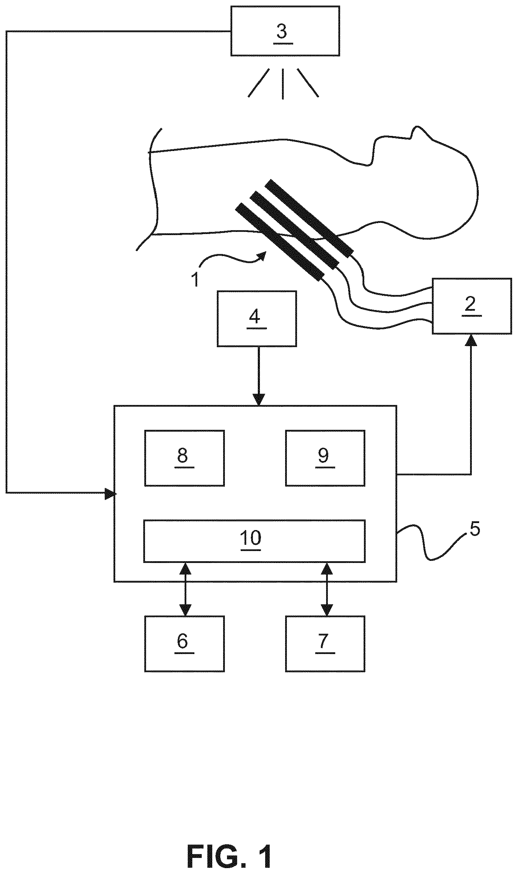

Fig. 1 schematically and exemplarily illustrates an embodiment of a system for delivering brachytherapy treatments to target structures within human or animal patient bodies. The target structures may particularly be tumors within certain regions of the bodies, such as the prostate or the female breast. In the exemplary embodiment shown in Fig. 1, the system is configured as a temporal brachytherapy system for delivering a high-dose rate (HDR) brachytherapy treatment or another form of temporal brachytherapy treatment.

In the brachytherapy system, a target structure is irradiated by means of radiation sources, which are temporarily placed in a treatment region in the vicinity of the target structure. The treatment may be delivered once or in plural fractions (i.e. radiation sources are placed in the treatment region several times).

The brachytherapy system comprises an applicator 1 for delivering the radiation sources to the treatment region. The radiation sources may particularly include radioactive particles emitting ionizing radioactive radiation for treating the target structure. The applicator 1 includes catheters for receiving the radiation sources. Via the catheters, the radiation sources can be delivered to the treatment region and hold at specific positions, which are also referred to as dwell positions and which usually correspond to the tips of the catheters of the applicator 1, for specific time periods, which are also referred to as dwell times. In the embodiment illustrated in Fig. 1, the radiation sources are automatically delivered into the applicator 1 from an afterloader device 2. In further embodiments, the radiation sources can likewise be delivered manually into the applicator 1.

Further, the system comprises an imaging device 3 that is configured to acquire images of the treatment region within the patient body. Preferably, the imaging device 3 is configured to generate three-dimensional images of the treatment regions. For this purpose, the imaging device 3 may employ any suitable imaging modality known to a person skilled in the art. Exemplary imaging modalities employed by the imaging device 3 include computed tomography (CT), ultrasound imaging or magnetic resonance imaging (MRI). In principle, it is also possible that the imaging device 3 is configured to acquire two-dimensional images of the treatment region by means of x-ray imaging, ultrasound imaging or another imaging technique. On the basis of the images, the anatomical configuration of the treatment region can be inspected and the location of the applicator 1, particularly the locations of the tips of the catheters of the applicator 1, in the treatment region can be determined, when images are acquired while the applicator 1 is positioned in the treatment region.

In some situations, the localization of the applicator 1 and of the tips of the catheters included therein on the basis of images may be inaccurate, e.g. when the catheter tips cannot be clearly identified in the images. In order to determine the positions with a higher accuracy, the system may additionally comprise a non-image based tracking device 4 for determining the position of the applicator 1 and the tips of the catheters included therein. The tracking device 4 may be configured in accordance with any suitable tracking technique known to a person skilled in the art. In one exemplary implementation, the tracking device 4 may be configured as an electromagnetic (EM) tracking apparatus. In this case, the tracking device 4 may generate a position varying electromagnetic field in the treatment region and the tips of the applicator catheters may be equipped with miniaturized electromagnetic field sensors, which are localized in the generated electromagnetic fields on the basis of field measurements carried out with the sensors.

The brachytherapy treatment is delivered in accordance with a treatment plan, which specifies the relevant treatment parameters particularly including the number of radiation sources and their dwell positions and dwell times and which is generated in a planning unit 5. The planning unit 5 may be configured as a computer device, such as, for example a personal computer, comprising a processing unit which executes a treatment planning software for generating treatment plans and for evaluating the treatment plans as will be described herein below. The planning unit 5 comprises a suitable interface for receiving images of the respective treatment region from the imaging device 3 and for receiving position information of the applicator determined using the tracking device 4. Further, the planning unit 5 comprises or is coupled to a user interface for interacting with a user (which may e.g. be a physician). The user interface may particularly comprise a display unit 6, such as a monitor, and input means 7 for performing user inputs, such as, for example a keyboard and/or a pointing device for navigating in a graphical user interface provided on the display unit 6.

In the planning unit 5, the treatment plan is determined on the basis of a clinical prescription for the patient, which may particularly specify treatment goals with respect to the target structure. These treatment goals may include the delivery of a certain minimum radiation dose to the target structure. In addition, treatment goals with respect to the OARs may be specified. These treatment goals may include the delivery of maximum radiation doses to be delivered to the OARs. Moreover, the treatment plan is determined on the basis of the positions of the target structure and the OARs in the treatment region, which are determined using an image of the treatment region, which may be acquired using the imaging device 3 and which is also referred to as planning image herein. In order to determine the positions of the target structure and OARs, delineations of the target structure and the OARs are determined in the planning image using a suitable delineation procedure, which may be a manual, semi-automatic or automatic delineation procedure.

In the process of generating the treatment plan, appropriate dwell positions in the treatment region may be determined in a positioning module 8 of the planning unit 5 on the basis of the planning image and the delineations of the target structure and the OARs. The dwell positions may be determined on the basis of the positions of the target structure and the OARs by applying a heuristic determination procedure. Known examples of such a procedure include the so-called k-means clustering procedure and the so-called centroidal Voronoi tessellation.

On the basis of the dwell positions, the treatment plan may then be further determined in a plan module 9 of the planning unit 4. In particular, the plan module 9 may determine the dwell times during which the treatment region is irradiated by means of the radiation sources located at the dwell positions and adds these dwell positions to the treatment plan.

The plan module 9 determines the dwell times in such a way that the treatment goals are fulfilled to the best possible extent. For this purpose, the plan module 9 may generate a set of constraints on the basis of treatment goals. The constraints correspond to requirements that the dose distribution should fulfill. Possible constraints particularly comprise the delivery of a maximum and minimum radiation dose to specific locations or regions within the treatment region. Minimum dose requirements usually relate to the target structure. So, a minimum radiation dose to be delivered to one or more locations or regions of the target structure may particularly be specified. Maximum dose requirements usually relate to the OARs. In this regard, a maximum radiation dose to be delivered to one or more locations or regions of the OARs may particularly be specified. In addition, further constraints may be defined, such as, for example, the delivery of a uniform dose distribution to a certain region of the treatment volume (which will usually be a region of the target structure).

On the basis of the generated constraints, an optimization problem may be formulated and at least approximately solved in order to determine the dwell times for the radiation sources. Hereby, the dwell times are determined in such a way that the distribution of the radiation dose delivered to the treatment region by means of the radiation sources positioned at the dwell positions fulfills the treatment goals to the best possible extent. Thus, the plan module 9 may effectively determine an optimized radiation dose distribution and related dwell times.

The optimization problem can be formulated and solved in a way known to a person skilled in the art. In one implementation, the set of constraints may be translated into a cost function and the optimization carried out in the plan module 9 involves a minimization of the cost function.

The cost function $F$ may comprise a collection of individual objective functions $f^k$, where each individual objective function $f^k$ represents one soft constraint. In one embodiment, the cost function $F$ may particularly correspond to a weighted sum of the objective functions $f^k$, i.e.

$$F(t) = \sum_{k=1}^{N} w^k f^k \,,$$

where $t$ is a vector quantity including the set of dwell times to be determined and the parameter $w^k$ denotes the weight of the objective function $f^k$. Due to the weighting, constraints having a higher weight are satisfied more likely than soft constraints having a lower weight, particularly in case such constraints are in conflict with each other. Hence, the weights are selected in accordance with the importance of the constraints with respect to the success of the treatment.

The cost function $F$ and the individual objective functions $f^k$ may particularly be functions of the dose distribution $d$ which is a vector quantity specifying the radiation dose $d_i$ absorbed by each volume element of the treatment region during the treatment, where the volume elements may correspond to the voxels in the image space of the planning image and where the dose distribution is a function of the dwell positions and the dwell times. More specifically, the radiation dose $d_i$ absorbed by the volume element $i$ of the treatment region may be linearly approximated on the basis of an influence matrix P in accordance with

$$d_i = \sum_j P_{ij} \cdot t_j$$

where $P_{ij}$ denotes the $i,j$-component of the influence matrix $P$ and $t_j$ denotes the dwell time of the radiation source at the dwell position $j$. Each component $P_{ij}$ of the influence

matrix quantifies the amount of dose absorbed by the volume element *i* per unit time due to emission from the radiation source at dwell position *j*. The influence matrix may be calculated on the basis of the dwell positions (determined as explained above), the anatomical configuration of the relevant region of the patient body and the known radiation intensity emitted by the radiation sources.

Each of the objective functions may include a penalty term, particularly a linear or quadratic penalty term, which penalizes deviations from desired dose values. As an example, an objective function representing a maximum/minimum radiation dose for a certain volume *V* may be given by

$$ f^k = \sum_{i \in V} g\left(d_i, d^k\right) \cdot \left[ \frac{d_i - d^k}{d^k} \right]^2 \cdot \Delta v_i \, , $$

where $g(d_i, d^k) = H(d_i - d^k)$ in case a maximum dose is specified and $g(d_i, d^k) = H(d^k - d_i)$ in case a minimum dose is specified. $\Delta v_i$ denotes the volume of the voxel i, $d_i = d_i(t)$ is the radiation dose delivered to the voxel i when the radiation parameters *t* are used, $d^k$ is the maximum/minimum radiation dose to be delivered to the volume *V,* and *H* is the Heaviside step function defined by

$$ H(x) = \begin{cases} 0, & x < 0 \\ 1, & x \geq 0 \end{cases} . $$

As said above, the dwell times to be included into the treatment plan may be determined by minimizing the cost function *F(t)* of the type described above, which is generated for the previously determined dwell positions, with respect to the dwell times.

In order to perform the minimization of the cost function *F(t),* the plan module 9 may particularly carry out an automatic numerical calculation. Optionally, it is also possible to carry out a user-guided iterative optimization procedure comprising several steps. In each step, the plan module 9 automatically calculates a version of the treatment plan by approximating a solution of the optimization problem. Then, the plan module 9 determines the dose distribution corresponding to this treatment plan and visualizes the dose distribution to the user of the planning unit 5. The user reviews the dose distribution to decide whether he/she is largely satisfied with the dose distribution. If this is the case in one step, the version of the treatment plan calculated in this step is used as the optimized treatment plan. If the user is not satisfied, the optimization problem is modified in accordance with changes specified by the user as a result of his/her review. Then, the plan module 9 calculates a new version of the treatment plan in the next step.

In principle, the plan module 9 can determine the dwell times in accordance with the aforementioned procedure on the basis of the nominal dwell positions as determined in the positioning module 8. In this case, the applicator 1 maybe inserted into the treatment region upon having determined the complete treatment plan including the dwell times. In the process of inserting the applicator 1, the tips of the catheters of the applicator 1 would be positioned at the determined nominal dwell positions. Thereupon, the radiation sources would be delivered to the dwell positions in accordance with the determined dwell times.

However, this procedure would be prone to errors because it may not be possible to position the catheter tips precisely at the determined dwell position. Therefore, the applicator 1 is usually inserted into the treatment region before the dwell times are determined. The insertion may be carried out in such a way that the tips of the catheters of the applicator 1 are positioned as closely as possible to the dwell positions determined in the positioning module 8 beforehand. Then, the dwell positions, which correspond to the positions of the tips of catheters of the applicator 1, may be determined and the dwell times may be calculated on the basis of the determined dwell positions rather than on the basis of the nominal positions as determined in the positioning module 8. Thus, the optimized treatment plan includes the dwell positions as determined upon the insertion of the applicator 1 into the treatment region. These positions are the planned dwell positions in this procedure. In order to establish these dwell positions, the positions of the tips of the catheters of the applicator 1 may be automatically or manually determined on the basis of an image of the treatment region including the inserted applicator 1, which may be acquired using the imaging device 3, and/or measured by means of the tracking device 4.

While the latter approach reduces errors resulting from an incorrect positioning of the applicator 1, it is still affected by errors or inaccuracies in the determination of the dwell positions. If the dwell positions are determined on the basis of an image of the treatment region such inaccuracies may occur when it is not possible to precisely identify the catheter tips in the image. In case the dwell positions are measured by means of the tracking device 4, such inaccuracies may result from the inherent measurement error of a tracking device 4. Moreover, an additional source of inaccuracies (in both aforementioned approaches) relates to the dwell times because the actual dwell times may deviate from their values as determined in the plan module 9 and included in the treatment plan. Such deviations may result from inaccuracies in the operation of the afterloader device 2 or from an inaccurate handling of the radiation sources in a manual insertion procedure for inserting the radiation sources into the applicator 1.

In view of these possible errors in the actual values of the treatment parameters, a robustness analysis may be carried out in which the possible effects of the errors on the distribution of the radiation dose delivered to the treatment region are determined and evaluated. In related embodiments of the system, the treatment plan determined in the plan module 9 is treated as a preliminary treatment plan and the robustness of the preliminary treatment plan against the aforementioned inaccuracies in the determination of the treatment parameters (particularly the dwell positions) and/or in setting the treatment parameters (particularly the dwell times) to the values included in the treatment plan may be determined in the system.

A treatment plan may considered to be sufficiently robust against such inaccuracies if the possible error values of the parameters only result in acceptable deviations of the delivered dose distribution from the optimized dose distribution determined in the plan module 9, which do not prevent that the treatment goals are fulfilled. On the other hand, a treatment plan may be considered to lack sufficient robustness if the possible error values of the treatment parameters may result in larger deviations from the optimized dose distribution so that the treatment goals will likely not be fulfilled.

If a sufficient robustness of the preliminary treatment plan is established in the robustness analysis, the brachytherapy treatment may be delivered on the basis of the preliminary treatment plan determined in the plan module 9. Thus, the preliminary treatment plan becomes the final treatment plan in this case. However, if the robustness analysis reveals a lack of sufficient robustness, the preliminary treatment plan may be further optimized to achieve a sufficient robustness against the inaccuracies. This process, which will be further explained herein below, is also referred to as robustness optimization herein.

In alternative embodiments, the treatment plan may be optimized with respect to its robustness against the aforementioned inaccuracies right away, i.e. without a preceding robustness analysis. These embodiments have the advantage that a more robust treatment plan is generated more quickly in situations in which the robustness analysis would reveal a lack of robustness of a conventionally generated preliminary treatment plan. On the other hand, the robustness optimization usually involves a very high computational complexity. Therefore, it may be advantageous to apply the robust optimization only in cases in which a conventionally determined preliminary treatment plan lacks sufficient robustness. In practical implementations, it may therefore be beneficial to perform the robustness optimization only upon a preceding robustness analysis of a preliminary treatment plan in case this analysis results in a determination that the preliminary treatment plan lacks sufficient robustness.

In order to carry out the robustness analysis for a preliminary treatment plan generated in the plan module 9 and in order to carry out the robustness optimization, the planning unit 5 may comprise a robustness evaluation unit 10.

In the robustness evaluation unit 10, the robustness analysis of a preliminary treatment plan may be performed with respect to all treatment parameters of the brachytherapy system or a subset of treatment parameters. The relevant treatment parameters in respect of which the robustness analysis is carried out are also referred to as perturbed parameters herein below. In one embodiment, these parameters (only) correspond to the dwell positions. Optionally, the dwell times may additionally be taken into consideration as perturbed parameters in the robustness analysis.

The robustness evaluation unit 10 may generate a set of perturbed parameter configurations, where each perturbed parameter configuration comprises a perturbed value of each perturbed parameter and further comprises the parameter values included in the preliminary treatment plan for all remaining treatment parameters. The perturbed parameter value of a specific treatment parameter deviate from the value of the parameter included in the preliminary treatment plan, which is also referred to as planned value herein, by an amount corresponding to an error value of the respective treatment parameter or less. The error value may correspond to the maximum deviation of the value of the parameter from the planned value due to inaccuracies in the determination of the planned value and/or in the setting of the parameter to the planned value. Likewise, the error values may be determined in another way and may correspond to the mean error in the determination of the planned value and/or in the setting of the parameter to the planned value, for example.

The error value may be pre-stored in the robustness evaluation unit 10 for each treatment parameter. The pre-stored value may be specified on the basis of past experience. As an alternative, the pre-stored value may be specified on the basis of the known (in)accuracy of the determination of the respective treatment parameter and/or in the setting of the treatment parameter to the planned value. For instance, in case of the dwell positions and a determination of the dwell positions using the tracking device 4, the pre-stored value may correspond to the known maximum error or mean error of the position measurement by means of the tracking device 4. As a further alternative, the error value may be specified by the user of the planning unit 5. This may particularly be useful for the error values for the dwell positions, in case the dwell positions are manually determined in an image of the treatment region including the applicator 1 by the user of the planning unit 5. In certain implementations, it may also be possible for the user of the planning unit 5 to select the error value of a treatment parameter from a manually input error value and one or more pre-stored error values. In case multiple error values are provided for a selection by the user, one value may be specified on the basis of past experience and a further value may be derived from the known (in)accuracy of the determination of the respective treatment parameter and/or in the setting of the treatment parameter to the planned value.

Within the value range limited by this error value, the perturbed parameter values may be generated randomly in the robustness evaluation unit 10 (where a random generation as understood herein also encompasses a generation on the basis of a pseudorandom procedure). Thus, each perturbed parameter configuration comprises one value of each perturbed parameter, which is randomly selected from a neighborhood of the planned value that is limited by the maximum error value. For instance, in order to determine a perturbed dwell position, the robustness evaluation unit 10 may select a random position within a spherical region around the planned dwell position, which corresponds to the perturbed value of the dwell position. The radius of the spherical region may correspond to the position error value. By means of the random generation of the perturbed values, it can be achieved that realistic error scenarios are generated, if a sufficiently large number of perturbed parameter configurations is determined.

The number of perturbed parameter configurations to be generated may be pre-stored in the robustness evaluation unit 10 or it may be specified by the user of the planning unit 5. In general, it is preferred to determine a larger number of perturbed configurations in order to increase the reliability of the perturbation analysis. However, a larger number of perturbed parameter configurations increases the computational complexity of the robustness analysis. Therefore, a compromise may be made. In this respect, an appropriate number of perturbed parameter configurations maybe between 20 and 300.

Upon having generated the perturbed parameter configurations, the robustness evaluation unit 10 may estimate a radiation dose distribution for each of the perturbed parameter configurations, which corresponds to the dose distribution that would be delivered to the treatment region during the treatment if the treatment was carried out on the basis of the respective parameter configuration. In order to calculate the dose distribution, the radiation dose $d_i$ absorbed by each volume element i of the treatment region may be linearly approximated on the basis of an influence matrix P as described above, i. e. in accordance with

$$d_i = \sum_j P_{ij} \cdot t_j .$$

Thus, for each perturbed parameter configuration the robustness evaluation unit 10 may calculate an influence matrix $P^k$ (where the upper index k denotes the perturbed parameter configuration) and may estimate a dose distribution on the basis of the matrix $P^k$ and on the basis of the dwell times included in the perturbed parameter configuration, which may correspond to the dwell times included in the optimized treatment plan determined in the plan module 9 or to perturbed dwell times.

When a perturbed parameter configuration only comprises perturbed dwell positions (and no disturbed dwell times), the radiation dose distribution resulting from the perturbed parameter configuration significantly deviates from the optimized dose distribution determined in the plan module 9 in a limited region surrounding the relevant (planned) dwell positions because each radiation source usually only has a significant influence on the radiation dose distribution in the limited region surrounding the related dwell position. For each dwell position, the size of this region depends on the dwell time for the radiation source at the respective dwell position, where a larger dwell time leads to a larger region. In order to estimate the dose distribution for a perturbed parameter configuration only including perturbed dwell positions, the robustness evaluation unit 10 may therefore newly calculate dose values only for volume elements located in regions surrounding the planned values of the relevant dwell positions. For the remaining sections of the treatment region outside the aforementioned regions, the dose values from the optimized dose distribution determined in the plan module 9 may be included in the estimate of the dose distribution for the perturbed parameter configuration. Hereby, the computational complexity can be significantly reduced. Further, this approach only requires a calculation of those matrix elements of the influence matrix pertaining to the respective perturbed parameter configuration, which relate to the volume elements included in the aforementioned limited regions. Therefore, the robustness evaluation unit 10 may only calculate these matrix elements and may not calculate the matrix elements relating to other volume elements.

The aforementioned regions may be essentially spherical and their sizes may be determined on the basis of the associated dwell times. In particular, the region associated with a radiation source at a certain dwell position and radiating during a certain dwell time may include the volume elements to which a dose value above a pre-determined threshold is delivered from that radiation source in accordance with the influence matrix (where the influence matrix generated on the basis of the planned dwell positions may be used for determining this region).

On the basis of the estimated dose distributions for the generated perturbed parameter configurations, the robustness of the preliminary treatment plan may further be determined in a user-assisted procedure. In exemplary implementations of this procedure, the robustness evaluation unit 10 may visualize the determined dose distributions for the perturbed parameter configurations to the user of the planning unit 5 at the display unit 6. By visually inspecting the dose distributions, the user may determine whether the possible error-induced deviations from the optimized dose distribution determined in the plan module 9 are acceptable or not.

In order to ease the assessment of the robustness of the preliminary treatment plan, the robustness evaluation unit 10 may additionally or alternatively determine one or more statistical features for each dose distribution and present these features to the user on the display unit in a suitable format.

The statistical features may particularly include one or more dose volume histograms (DVHs), where each DVH may illustrates which fractions of a certain volume, such as the target structure, absorbs at least a certain radiation dose. More specifically, such a DVH may be plotted with radiation doses D on the horizontal axis and fractions RV of the relevant volume on the vertical axis, where a value provided in the diagram specifies the fraction of the volume which absorbs at least the associated dose value. Such a DVH curve for the target structure and/or one or more OARs may be generated for each dose distribution and the curves may be presented in a single diagram so that the user can inspect the range of DVH values resulting from the perturbed parameter configurations.

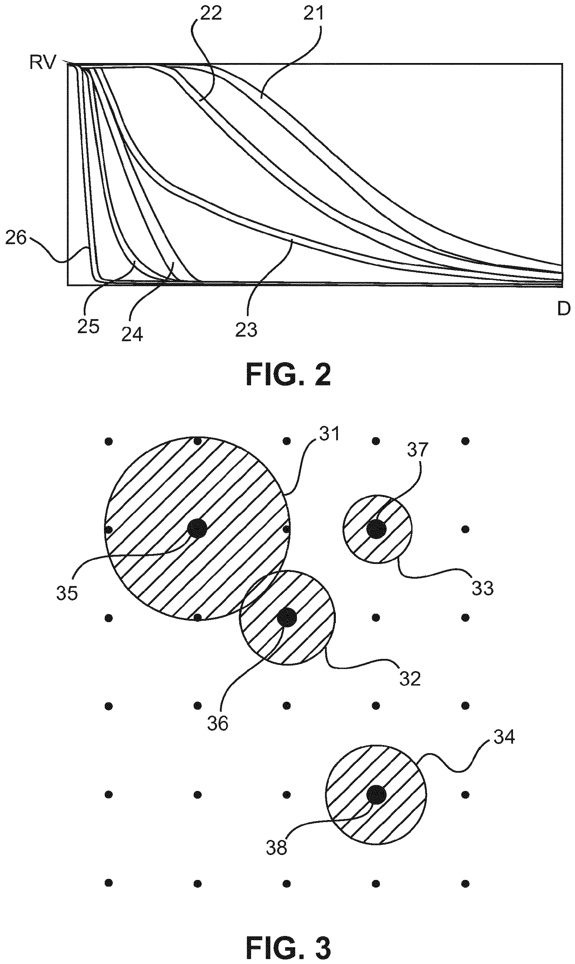

An exemplary diagram that shows DVH curve sets 21-26 for a target structure (curve set 21) and several OARs is illustrated in Fig. 2. Each set of curves comprises the curves derived from the radiation dose distributions pertaining to perturbed parameter configurations. On the basis of such a diagram, the user of the planning unit 5 may judge whether or not the sets of curves are within acceptable ranges and, thus, whether or not the treatment plan is sufficiently robust.

Moreover, the robustness evaluation unit 10 may calculate mean values and standard deviations for specific statistical parameters, which may relate to the DVHs. For instance, the robustness evaluation unit 10 may calculate the mean values and the standard deviations of minimum or maximum dose values absorbed by certain percentages of a specific region, such as the target structure and/or an OAR. On the basis of these parameters, the user of the planning unit 5 may likewise judge whether or not the treatment plan is sufficiently robust. For instance, the robustness evaluation unit 10 may calculate the mean value and the standard deviation of the dose level absorbed by 95% of the volume of the target structure, as this value is typically one indicator of the effectiveness of the radiation treatment.

If the user of the planning unit 5 determines on the basis of the information provided by the robustness evaluation unit 10 that the preliminary treatment plan is sufficiently robust, the treatment may be delivered on the basis of the preliminary treatment plan as determine in the plan module 9. Thus, the preliminary treatment plan becomes the final treatment plan for carrying out the treatment.

If the user of the planning unit 5 determines that the preliminary treatment plan is not sufficiently robust, a robustness optimization of the treatment plan may be carried out in a robustness evaluation unit 10 of the planning unit 5. In particular, the robustness optimization may be carried out in case the perturbed parameter configurations only include perturbed dwell positions. This case will also be assumed in the following description of embodiments of the robustness optimization.

The robustness optimization may be carried out on the basis of the perturbed parameter configurations to determine a new optimized set of dwell times. In one implementation, the robustness evaluation unit 10 may generate a cost function $F_k(t)$ for each perturbed parameter configuration $k$ and may formulate a min-max problem on the basis of the cost functions, which is then solved in the robustness optimization unit 11. In one implementation, the robustness optimization unit minimizes the maximum of all cost functions $F_k(t)$ with respect to the dwell times $t$. Thus, the robustness optimization unit 11 may determine the

$$\min_t \left[ \max_k F_k(t) \right]$$

The individual cost functions $F_k$ for the perturbed parameter configurations may be generated as described above in connection with the determination of the dwell times in the plan module 9. Thus, each cost function $F_k$ may particularly be a function of the dwell times t and the influence matrix $P^k$ pertaining to the respective perturbed parameter configuration $k$. When the robustness optimization is carried out, these matrices may have been determined by the robustness evaluation unit 10 as explained above and the determined matrices may be re-used. If the matrices are not available, they may be newly calculated in the robustness evaluation unit 10 for carrying the robustness optimization.

In the determination of the influence matrices for generating the cost functions, the aforementioned procedure may be applied in one embodiment, in which only those matrix elements are newly calculated which relate to volume elements located in regions surrounding the planned values of the dwell positions for which perturbed values are included in the respective perturbed parameter configuration for which the influence matrix is calculated. Hence, the robustness evaluation unit 10 may only calculate these matrix elements and may take the other matrix elements from the influence matrix associated with the planned values of the dwell positions. Hereby, the computational complexity can again be reduced.

Upon having determined the optimized treatment plan in the robustness optimization process of the aforementioned embodiment, the treatment may be delivered on the basis of this optimized treatment plan. It has been found that the optimized plan calculated on the basis of the aforementioned embodiment has a significantly increased robustness against errors of the dwell positions that may result from inaccuracies in the determination of the dwell positions as explained above.

In further embodiments, the robustness analysis and optimization may further include an estimation of the influences of the individual dwell positions on deteriorations of the radiation dose distribution compared with the optimized radiation dose distribution determined in the plan module 9 and the robustness optimization may be performed on the basis of this estimation.

As explained above, each radiation source usually only has a significant influence on the radiation dose distribution in a limited region surrounding the related dwell position, which is also referred to as influence region in the following. The size of this influence region depends on the dwell time for the radiation source and on the related elements of the influence matrix. If a radiation source at a certain dwell position only has a low influence on the radiation dose distribution (i.e. if the radiation source has a small influence region), perturbed values of this dwell position may not be considered in the robustness optimization. Rather, the values of this dwell position may be set to the planned value in all perturbed configurations for carrying out the robustness optimization. Hereby, the computational complexity of the robustness optimization can be reduced, particularly in case dose values are only newly calculated in the neighborhoods of the dwell positions for which perturbed values are generated, as explained above.

On the other hand, one or more radiations source(s) having a greater influence on the radiation dose distribution (i.e. radiation sources having a larger influence region) may be excluded from the treatment plan so that it/they is/are not used in the later treatment. In this case, the robust optimization may be carried out on the basis of the remaining subset of dwell positions or one or more dwell position(s) could be added to replace the excluded dwell position(s) and the robustness optimization may be carried out on the basis of remaining subset of the previous dwell positions and on the basis of the new dwell position(s). In so doing, perturbed values of the new dwell position(s) may be included in the perturbed parameter configurations. Moreover, the influence matrix may be adapted to the new dwell position(s) and its/their perturbed value(s). By excluding the radiation source(s) having a greater influence on the radiation dose distribution, it may be possible to increase the likelihood that a significantly more robust treatment plan can be generated in the robustness optimization procedure.

The dwell positions that are to be set to their planning values in the robustness optimization and/or the dwell positions to be excluded from the treatment plan may be selected by the user of the planning unit 5. In particular, the user may perform the selection on the basis of a visualization of the influence regions of the radiation sources. An example of a corresponding visualization is shown in Fig. 3, which illustrates the influence regions 31-34 of radiation sources located at dwell positions 35-38.

In the way described above, a robustness optimization of a preliminary treatment plan may be carried out by newly optimizing the dwell times on the basis of the perturbed parameter configurations. As explained, the optimization may be carried out if the preceding robustness analysis has revealed a lack of sufficient robustness of the preliminary treatment plan. As an alternative, the robustness evaluation unit 10 may perform the robustness optimization right away on the basis of the planned dwell positions determined in the positioning module 8. In this embodiment, the dwell times are calculated for the first time using the robustness optimization procedure explained above.

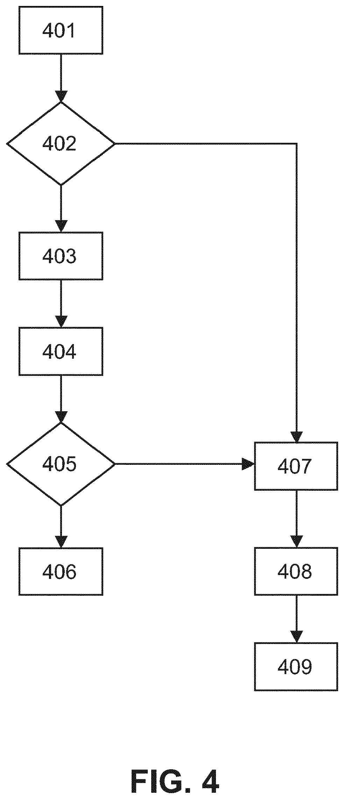

In Fig. 4, exemplary steps of embodiments of a procedure carried out in the robustness evaluation unit 10 are illustrated: In step 401, the robustness evaluation unit 10 may obtain a set of planned dwell positions. The planned dwell positions may be included in a preliminary treatment plan generated in the plan module 9, which also includes planned dwell times, and the robustness of the preliminary treatment plan may be analyzed. Alternatively, it may also be possible that the robustness evaluation unit 10 receives the planned dwell positions in order to directly generate a treatment plan in the robustness optimization procedure on the basis of the planned dwell position.

If both options are possible in the system (which does not necessarily have to be the case), the system may check in step 402 whether the planned dwell positions are included in a preliminary treatment plan, the robustness of which is to be analyzed, or whether the planned dwell positions are provided for the generation of a treatment plan.

If the planned dwell positions are included in a preliminary treatment plan, the robustness of which is to be analyzed, the robustness evaluation unit 10 may generate perturbed parameter configurations including perturbed dwell positions and the planned dwell times in step 403. Alternatively, the perturbed parameter configurations may also include perturbed dwell times. Thereupon, the robustness evaluation unit 10 may estimate radiation dose distributions for the perturbed parameter configurations and the robustness of the preliminary treatment plan may be analyzed on the basis of the estimated dose distributions as explained above (step 404). Then it is checked whether the preliminary treatment plan is sufficiently robust in step 405. If so, the treatment is delivered on the basis of the preliminary treatment plan in step 406.

If it is determined in step 405 that the preliminary treatment plan lacks sufficient robustness, a further perturbed parameter configuration is generated in step 407 which includes the perturbed dwell positions (or a subset thereof) and does not include specific values of the dwell times. Optimized dwell times are then determined in accordance with the robustness optimization procedure in step 408. As result of this process, an optimized treatment plan is provided which includes the planned dwell positions (or the relevant subset thereof) and the optimized dwell times, and the treatment may be delivered using this treatment plan in step 409.

If it is determined in step 402 that the planned dwell positions are provided for the generation of a treatment plan, the robustness evaluation unit 10 may directly proceed with step 407 explained above in order to determine a treatment plan in accordance with the robust optimization procedure.

Variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

**Claims**

1. A system for assisting in planning a radiation therapy treatment of a target structure in a region of a patient body, the treatment being delivered on the basis of a treatment plan including error-prone parameter values of first treatment parameters for controlling the delivery of the radiation, wherein the system comprises a robustness evaluation unit (10) configured to:

    - obtain planned parameter values of the first treatment parameters,
    - generate perturbed parameter configurations including perturbed values of the first treatment parameters, the perturbed parameter values deviating from the planned parameter values by possible error values of the first treatment parameters occurring during the treatment,
    - estimate for each perturbed parameter configuration a radiation dose distribution resulting from a treatment delivered on the basis of the perturbed parameter configuration and/or
    - determine the treatment plan on the basis of the perturbed parameter configurations.

2. The system as defined in claim 1, wherein the radiation is emitted by radiation sources located within the patient body at dwell positions during dwell times and wherein the first treatment parameters correspond to the dwell positions and/or the dwell times.

3. The system as defined in claim 1, wherein the perturbed parameter values deviate from the planned parameter values by a predetermined maximum amount or less.

4. The system as defined in claim 1, wherein the perturbed parameter values are generated randomly.

5. The system as defined in claim 1, wherein the planned values of the first treatment parameters are included in a preliminary treatment plan and wherein each of the radiation dose distributions estimated by the robustness evaluation unit (10) for a perturbed parameter configuration results from the preliminary treatment plan if the planned values of the first treatment parameter are replaced by the perturbed values of the first treatment parameters included in the respective perturbed parameter configuration.

6. The system as defined in claim 1, being further configured to visualize the radiation dose distributions estimated for the perturbed parameter configurations to a user of the system and/or to determine at least one statistical feature from the radiation dose distributions.

7. The system as defined in claim 2, wherein the planned values of the first treatment parameters are included a preliminary treatment plan, which corresponds to a planned radiation dose distribution, and wherein the robustness evaluation unit (10) is configured to estimate the radiation dose distribution for each perturbed parameter configuration by calculating dose values for volume elements of the treatment region in regions surrounding the dwell positions, the sizes of the regions being determined on the basis of the dwell times, and by taking dose values for volume elements of the treatment region outside the regions from the planned radiation dose distribution.

8. The system as defined in claim 1, wherein the treatment plan further includes parameter values of second treatment parameters and wherein the robustness evaluation unit is configured to generate the treatment plan by optimizing the parameters values of the second treatment parameters on the basis of the perturbed parameter configurations.

9. The system as defined in claim 8, wherein the radiation is emitted by radiation sources located within the patient body at a dwell positions during a dwell times and wherein the first treatment parameters correspond to the dwell positions and the second treatment parameters correspond to the dwell times.

10. The system as defined in claim 1, wherein the robustness evaluation unit (10) is configured to generate a set of cost functions comprising one cost function for each of at least some of the perturbed parameter configurations and to generate the treatment plan on the basis of the set of cost functions.

11. The system as defined in claim 8 or 9, wherein the robustness evaluation unit (10) is configured to generate the treatment plan by determining the

$$\min_{t} \left[ \max_{k} F_k(t) \right]$$

where $F_k$ denotes the cost function for the $k$-th perturbed parameter configuration and $t$ denotes a set of parameter values of the second treatment parameters.

12. The system as defined in claim 1, wherein the generated treatment plan only includes parameter values of a subset of the first treatment parameters and wherein the subset does not include values of one or more first treatment parameters having a greater influence on the radiation dose distribution corresponding to the treatment plan than other first treatment parameters.

13. The system as defined in claims 9 and 12, wherein the influence of a dwell position of a radiation source on the radiation dose distribution is determined on the basis of the dwell time of the radiation source.

14. A method for assisting in planning a radiation therapy treatment of a target structure in a region of a patient body, the treatment being delivered on the basis of a treatment plan including error-prone parameter values of first treatment parameters for controlling the delivery of the radiation, wherein the method comprises:

- obtaining (401) planned parameter values of the first treatment parameters,
- generating (403; 407) perturbed parameter configurations including perturbed values of the first treatment parameters, the perturbed parameter values deviating from the planned parameter values by possible error values of the first treatment parameters occurring during the treatment,
- estimating (404) for each perturbed parameter configuration a radiation dose distribution resulting from a treatment delivered on the basis of perturbed parameter configuration and/or
- determining (408) the treatment plan on the basis of the perturbed parameter configurations.

15. A computer program comprising program code for instructing a computer device to perform a method as defined in claim 14 when the program code is executed in the computer device.

**FIG. 1**

**FIG. 2**

**FIG. 3**

FIG. 4

Europäisches
Patentamt

European
Patent Office

Office européen
des brevets

## EUROPEAN SEARCH REPORT

Application Number

EP 17 19 4553

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIROYUKI OKAMOTO ET AL: "Dose error from deviation of dwell time and source position for high dose-rate 192Ir in remote afterloading system", JOURNAL OF RADIATION RESEARCH, vol. 55, no. 4, 23 February 2014 (2014-02-23), pages 780-787, XP055458412, JP ISSN: 0449-3060, DOI: 10.1093/jrr/rru001 | 1-3,5,6, 8-15 | INV. A61N5/10 |
| A | * the whole document * | 4,7 | |
| X | P Manser ET AL: "Calculation of uncertainties in brachytherapy treatment planning", , 1 January 2008 (2008-01-01), XP055458419, Retrieved from the Internet: URL:http://www.ssrpm.ch/old/2008/Manser-82.pdf [retrieved on 2018-03-12] * the whole document * | 1,2,14, 15 | |
| X | WO 2017/037060 A1 (KONINKLIJKE PHILIPS NV [NL]) 9 March 2017 (2017-03-09) * abstract * * page 10, line 5 - page 12, line 5 * | 1,14,15 | TECHNICAL FIELDS SEARCHED (IPC) A61N |
| A | US 2003/065260 A1 (CHENG GANG [US] ET AL) 3 April 2003 (2003-04-03) * abstract * | 1-15 | |

The present search report has been drawn up for all claims

| Place of search | Date of completion of the search | Examiner |
|---|---|---|
| Munich | 12 March 2018 | Grochol, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 4553

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2018

| Patent document cited in search report | | Publication date | Patent family member(s) | | Publication date |
|---|---|---|---|---|---|
| WO 2017037060 | A1 | 09-03-2017 | NONE | | |
| US 2003065260 | A1 | 03-04-2003 | AU | 5155401 A | 12-11-2001 |
| | | | CA | 2407577 A1 | 08-11-2001 |
| | | | EP | 1278560 A2 | 29-01-2003 |
| | | | US | 6438401 B1 | 20-08-2002 |
| | | | US | 2003065260 A1 | 03-04-2003 |
| | | | WO | 0182995 A2 | 08-11-2001 |

EPO FORM P0459

For more details about this annex : see Official Journal of the European Patent Office, No. 12/82